# EUROPEAN PATENT APPLICATION

(11) **EP 4 706 754 A1**
(43) Date of publication of application: **11.03.2026**
(21) Application number: 24800221.4
(22) Date of filing: 30.04.2024
(51) Int. Cl.: A61N 7/02, A61N 7/00

(54) **ULTRASONIC WAVE GENERATING DEVICE HAVING PLURALITY OF ULTRASONIC TRANSDUCERS, AND ULTRASONIC TREATMENT HANDPIECE INCLUDING SAME**

(30) Priority: 03.05.2023 KR 20230057463
(71) Applicant: CLASSYS INC., Seoul 06220 (KR)
(72) Inventor: PARK, Su Man, Yongin-si Gyeonggi-do 16821 (KR); PARK, Si Hyung, Seoul 08329 (KR); CHOI, Joon Hyun, Seoul 06273 (KR)
(74) Representative: Wörz, Volker Alfred
(86) International application number: PCT/KR2024/005855
(87) International publication number: WO 2024/228538

(57) **Abstract**

The present invention relates to an ultrasonic wave generating device having a plurality of ultrasonic transducers and an ultrasonic treatment handpiece including the same. The prevent invention comprises: a cartridge housing unit having, on a lower surface, a window part, which is in contact with the skin and through which ultrasonic waves pass; a plurality of ultrasonic transducer units which are located in the cartridge housing unit and generate ultrasonic waves; a rotary mounting unit which is rotatably positioned inside the cartridge housing unit and on which the plurality of ultrasonic transducer units are mounted; and a rotary motor unit for rotating the rotary mounting unit. The prevent invention can selectively generate a plurality of ultrasonic waves by using the plurality of ultrasonic transducers, and can focus the respective ultrasonic waves so that the respective ultrasonic waves penetrate the skin to a different depth or individually control the intensities of the ultrasonic waves to achieve an ultrasonic treatment or skin care effect according to the skin condition of a patient. The present invention can also enhance the ultrasonic treatment or skin care effect.

## Description

### [Technical Field]

The present disclosure relates to an ultrasonic wave generating device having a plurality of ultrasonic transducers and an ultrasonic treatment handpiece including the same. More particularly, the present disclosure relates to an ultrasonic wave generating device having a plurality of ultrasonic transducers that can move the focal points of ultrasonic waves in a plane while rotating the plurality of ultrasonic transducers at a constant radius, and an ultrasonic treatment handpiece including the ultrasonic wave generating device.

### [Background Art]

In recent years, as dietary habits have become more Westernized, obesity has rapidly increased and has become the main factor that harms public health and beauty, and various diet programs and ultrasonic obesity treatment devices for treating obesity have been developed and are widely used.

High-Intensity Focused Ultrasound (HIFU) technology for obesity treatment was originally used for the purpose of anticancer treatment to destroy cancer cells by selectively coagulating internal organ tumors at high temperatures in a non-invasive manner. However, Solta Medical in the United States was the first to develop a device called Liposonix, which is equipped with HIFU for the purpose of treating abdominal obesity in the human body.

The process of fat destruction using HIFU is that when focused ultrasonic waves are concentrated at a specific point in fat cells, the temperature of the tissue instantaneously rises to 65°C to 100°C, thereby causing the tissue to be destroyed.

Unlike other dermatological equipment such as laser and RF equipment, HIFU equipment induces coagulation necrosis of fat by non-invasively concentrating HIFU energy on a selected area without causing any damage to the skin surface. The necrotic fat cells are naturally removed by the body's repair mechanism for damaged parts.

An example of a known ultrasonic obesity treatment device is Korean Patent No. 10-1365946 (published on February 24, 2014), titled "High Intensity Focused Ultrasound Generating Device for The Deduction of Fat Tissue".

The "High Intensity Focused Ultrasound Generating Device for The Deduction of Fat Tissue" enables ultrasonic waves to penetrate the skin by moving a transducer to a desired point in the X-axis and Y-axis directions and then pivoting it around the axes.

However, the "High Intensity Focused Ultrasound Generating Device for The Deduction of Fat Tissue" has a problem in that when ultrasonic waves are provided, they are delivered in an curved (arc) shape due to the characteristic of the pivot mechanism, so the energy delivered to the skin decreases and the focal depth changes toward the periphery, which results in uneven treatment.

In order to solve such a problem, the present applicant has proposed, in Korean Patent No. 10-1649899, titled "Ultrasonic Apparatus for Treatment," a structure that improves treatment performance by including a focal point rotating unit for circularly moving the focus of ultrasonic waves, which are generated from an ultrasonic generator, in the same plane, forming the focus of the ultrasonic waves in a circular shape having a predetermined radius at a uniform depth in the skin, and uniformly and evenly applying energy within the radius.

However, Korean Patent No. 1649899, titled "Ultrasonic Apparatus for Treatment," has a structure in which a plurality of protruding members protruding at different heights are brought into contact with the top of the ultrasonic generator, and thus has a limitation in reducing the size and also has a problem in that it is restricted in stably moving the focus of the ultrasonic waves, generated from an ultrasonic transducer unit, along a circular path in the same plane.

Further, in Korean Patent No. 1649899, titled "Ultrasonic Apparatus for Treatment", since the ultrasonic generator is rotated in a tilted state, ultrasonic waves are generated diagonally. As a result, the focal point of the ultrasonic waves is formed diagonally on the skin, so there is a problem in that it is difficult to uniformly transmit the intensities of the ultrasonic waves, i.e., the output of the ultrasonic waves, to the skin and it is difficult to increase the radius of the circle formed on the plane.

Further, Korean Patent No. 1649899, titled "Ultrasonic Apparatus for Treatment" has a structure in which a plurality of protruding members protruding at different heights are brought into contact with the top of the ultrasonic generator and the ultrasonic generator is rotated in a tilted state, so it has a problem in that the structure is complicated, the manufacturing cost increases, and a large load is required to rotate the ultrasonic generator due to torsional moment.

Korean Patent No. 10-1365946 (published on February 24, 2014), titled "High Intensity Focused Ultrasound Generating Device for The Deduction of Fat Tissue", and Korean Patent No. 10-1649899, titled "Ultrasonic Apparatus for Treatment", each include a single ultrasonic transducer, so the depth to which the ultrasonic focus reaches is limited to a single depth, whereby there is a problem in that the effect of ultrasonic treatment or skin care is limited it is difficult to achieve an ultrasonic treatment or skin care effect according to the skin condition of patients.

### [Disclosure]

### [Technical Problem]

An objective of the present disclosure is to provide an ultrasonic wave generating device having a plurality of ultrasonic transducers that can selectively generate a plurality of ultrasonic waves through the plurality of ultrasonic transducers and can enable focal points of the respective ultrasonic waves to penetrate to different depths in the skin, or can achieve an ultrasonic treatment or skin care effect according to the skin condition of patients by individually controlling the intensities of the ultrasonic waves, and an ultrasonic treatment handpiece including the ultrasonic wave generating device.

Further, another objective of the present disclosure is to provide an ultrasonic wave generating device that can enable ultrasonic waves to evenly and uniformly penetrate the skin by moving the focal points of the ultrasonic waves along circular paths at a uniform depth in the skin, and an ultrasonic treatment handpiece including the ultrasonic wave generating device.

Further, another objective of the present disclosure is to provide an ultrasonic wave generating device that makes it possible to easily adjust the output of ultrasonic waves by moving the focal points of the ultrasonic waves along circular paths in the same plane by rotating a plurality of ultrasonic transducer units at an offset position on a plane, that makes it possible to increase the radii of movement of the focal points, and minimizes the load on a motor during operation, and an ultrasonic treatment handpiece including the ultrasonic wave generating device.

Another objective of the present disclosure is to provide an ultrasonic wave generating device that makes it possible to reduce the size of an ultrasonic treatment handpiece by simplifying the structure for moving the focus of ultrasonic waves, generated from an ultrasonic transducer unit, along circular paths in the same plane, and enables ultrasonic treatment on localized areas of the patient's skin, such as under the eyes, and an ultrasonic treatment handpiece including the ultrasonic wave generating device.

### [Technical Solution]

In order to achieve the objectives described above, an embodiment of an ultrasonic wave generating device having a plurality of ultrasonic transducers according to the present disclosure includes: a cartridge housing having, on a bottom thereof, a window configured to come into contact with a skin and allow ultrasonic waves to pass through; a plurality of ultrasonic transducer units disposed in the cartridge housing and configured to generate ultrasonic waves; a rotary mount on which the plurality of ultrasonic transducer units are mounted and which is rotatably disposed in the cartridge housing; and a rotary motor unit configured to rotate the rotary mount.

In the present disclosure, the rotary motor unit may include a rotary shaft mounted on the rotary mount and connected to the rotary motor unit, and the plurality of ultrasonic transducer units may be mounted on the rotary mount at positions spaced apart from and eccentric to a rotation center of the rotary mount.

In the present disclosure, the rotary mount may include a plurality of mounting arm members disposed radially from a rotation center to which the rotary shaft is connected.

In the present disclosure, all of the plurality of ultrasonic transducer units may have structures that position focal points of ultrasonic waves at the same depth, or at least any one of the plurality of ultrasonic transducer units may have a structure that positions the focal point of an ultrasonic wave at a different depth.

In the present disclosure, the plurality of ultrasonic transducer units all may disposed to be spaced apart from the rotation center of the rotary mount by the same distance, or at least any one of the plurality of ultrasonic transducer units may be disposed to be spaced apart from the rotation center of the rotary mount by a different distance.

In the present disclosure, the ultrasonic transducer unit may be rotated around the rotary shaft while an ultrasonic generation surface is positioned horizontally, thereby moving a focal point of the ultrasonic wave generated from the ultrasonic transducer unit along a circular path in the same plane.

An embodiment of the ultrasonic wave generating device having a plurality of ultrasonic transducer according to the present disclosure may further include: a plurality of power supply protruding terminal units protruding upward from the rotary mount and configured to supply power to the plurality of ultrasonic transducer units; and a power supply board spaced above the rotary mount in the cartridge housing and to which the plurality of power supply protruding terminal units are connected.

In the present disclosure, a ring-shaped electrode pattern connected through a power cable to receive power may be disposed on a bottom of the power supply board, and the power supply protruding terminal unit may move along a circular path along the ring-shaped pattern while elastically in contact with the ring-shaped electrode pattern.

In the present disclosure, the cartridge housing may have a sealed internal structure and may be filled with an ultrasonic transmission medium, and the ultrasonic transmission medium may be an insulating liquid.

An embodiment of the ultrasonic wave generating device having a plurality of ultrasonic transducer according to the present disclosure may further include: a board position-fixing board positioned between a top of the cartridge housing and the power supply board and configured to support the position of the power supply board; a plurality of first board fixing portions having both ends fixed to the top of the cartridge housing and the board position-fixing board and configured to support the position of the board position-fixing board; and a plurality of second board fixing portions having both ends fixed to the board position-fixing board and the power supply board and configured to support the position of the power supply board.

In the present disclosure, the plurality of first board fixing portions and the plurality of second board fixing portions may be formed in a hollow tubular shape, and power cables connecting the ring-shaped electrode pattern and a first main power supply terminal protruding upward from the cartridge housing may be disposed inside the first and second board fixing portions.

The ultrasonic wave generating device may further include an ultrasonic transducer moving unit disposed under the rotary mount and configured to adjust the distance between the rotation center of the rotary mount and the ultrasonic transducer units by linearly moving the ultrasonic transducer unit.

In the present disclosure, the mounting arm members may include: a first arm member connected to the rotary shaft; a second arm member rotatably hinged to the first arm member and on which the ultrasound transducer units are mounted; and an arm rotation motor configured to rotate the second arm member in a plane about the hinge.

Further, in order to achieve the objectives described above, an embodiment of a therapeutic ultrasonic wave generating device according to the present disclosure includes: a body housing connected to a control body through a handpiece cable assembly; and an ultrasound generating device detachably coupled to the body housing, wherein the ultrasound generating device includes an embodiment of the ultrasonic wave generating device having a plurality of ultrasonic transducers according to the present disclosure.

### [Advantageous Effects]

The present disclosure selectively generates a plurality of ultrasonic waves using a plurality of ultrasonic transducers, and allows the focal points of ultrasonic waves to penetrate to different depths inside the skin or individually controls the intensities of ultrasound waves, thereby having the effects of achieving an ultrasonic treatment or skin care effect in accordance with the skin conditions of patients and enhancing the ultrasound treatment or skin care effect.

Further, the present disclosure applies energy uniformly and evenly to a treatment area by moving the focal points of ultrasonic waves in a plane at a uniform depth inside the skin, and applies energy uniformly and evenly within a predetermined radius by forming the focal points of the ultrasonic waves at a uniform depth inside the skin in a circular shape having the radius, thereby having an effect of improving treatment performance.

The present disclosure can further improve the treatment effect by moving the focal points of ultrasonic waves along circular paths in a plane by rotating the ultrasonic transducer units at offset positions in the plane to make it easy to adjust the output of ultrasonic waves, and has an effect of increasing the versatility of the treatment by expanding the range of the movement radius of the focal points.

Further, the present disclosure has the effect of minimizing the load on the motor and reducing manufacturing costs by simplifying the structure for moving the focal points of the ultrasonic waves generated by the plurality of ultrasonic transducer units along circular paths in the same plane.

### [Description of Drawings]

FIG. 1 is a perspective view showing an embodiment of an ultrasonic treatment handpiece according to the present disclosure.
FIG. 2 is an exploded perspective view showing an embodiment of the ultrasonic treatment handpiece according to the present disclosure.
FIG. 3 is a cross-sectional view showing an embodiment of an ultrasonic wave generating device having a plurality of ultrasonic transducers according to the present disclosure.
FIG. 4 is a partial enlarged bottom perspective view showing an embodiment of the ultrasonic wave generating device having a plurality of ultrasonic transducers according to the present disclosure.
FIG. 5 is an enlarged view of the portion A of FIG. 4.
FIG. 6 is a cross-sectional view showing another embodiment of the portion A of FIG. 4.
FIG. 7 is a cross-sectional view showing another embodiment of the ultrasonic wave generating device having a plurality of ultrasonic transducers according to the present disclosure.
FIG. 8 is a cross-sectional view showing another embodiment of the ultrasonic wave generating device having a plurality of ultrasonic transducers according to the present disclosure.

### Description of Reference Numerals

| | | | |
|---|---|---|---|
| 100 : | body housing | 101 : | cartridge locking portion |
| 110 : | handle | 111 : | handle bridge |
| 112 : | handle body | | |
| 200 : | cartridge housing | 201 : | upper housing |
| 201a : | cartridge board cap | 202 : | housing-sealing |
| 203 : | window | 210 : | medium supply protruding tube |
| 220 : | medium discharge protruding tube | 300 : | ultrasonic transducer unit |
| 400 : | rotary motor unit | 410 : | shaft adapter portion |
| 420 : | rotary shaft | 421 : | connecting shaft portion |
| 500 : | rotary mount | 510 : | mounting arm member |
| 511 : | first arm member | 512 : | second arm |
| | | | member |
| 513 : | arm rotation motor | | |
| 600 : | power supply protruding terminal unit | 600a : | negative protruding terminal unit |
| 600b | : positive protruding terminal unit | 610 : | jig pin member |
| 620 : | pin insertion member | 630 : | pin support spring member |
| 700 : | power supply board | 710: | ring-shaped electrode pattern |
| 711 : | first electrode pattern | 712 : | second electrode pattern |
| 800 : | board position-fixing board | 810 : | first board fixing portion |
| 820 : | second board fixing portion | 900 : | ultrasonic transducer moving unit |
| 1000 : | control body | | |

### [Mode of Invention]

Hereafter, the present disclosure is described in more detail.

Exemplary embodiments of the present disclosure are described hereafter in detail with reference to the accompanying drawings. Before describing the present disclosure, it should be noted that the terms or terminologies used the specification and the claims should not be construed as being limited to common meanings or the meanings in dictionaries. Therefore, the configurations described in the embodiments and drawings of the present disclosure are merely most preferable embodiments but do not represent all of the technical spirit of the present invention. Thus, it should be understood that the present disclosure should be construed as including all the changes, equivalents, and substitutions included in the spirit and scope of the present disclosure at the time of filing this application.

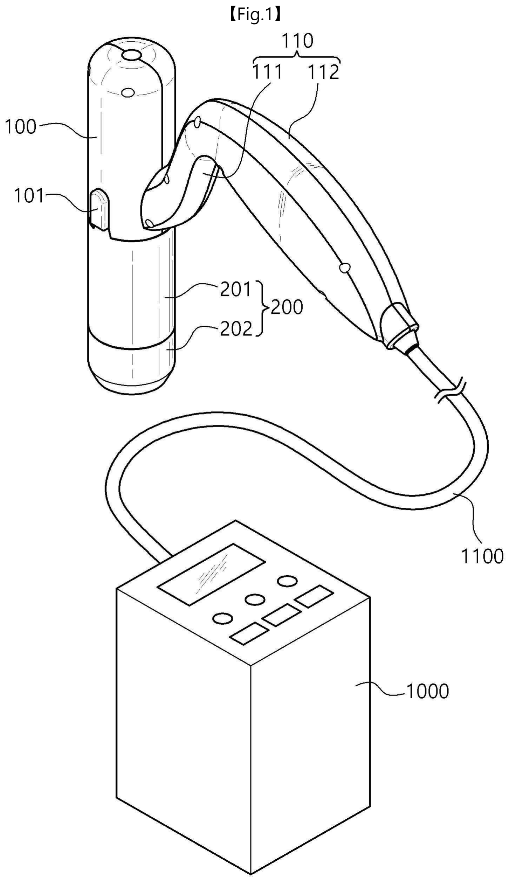

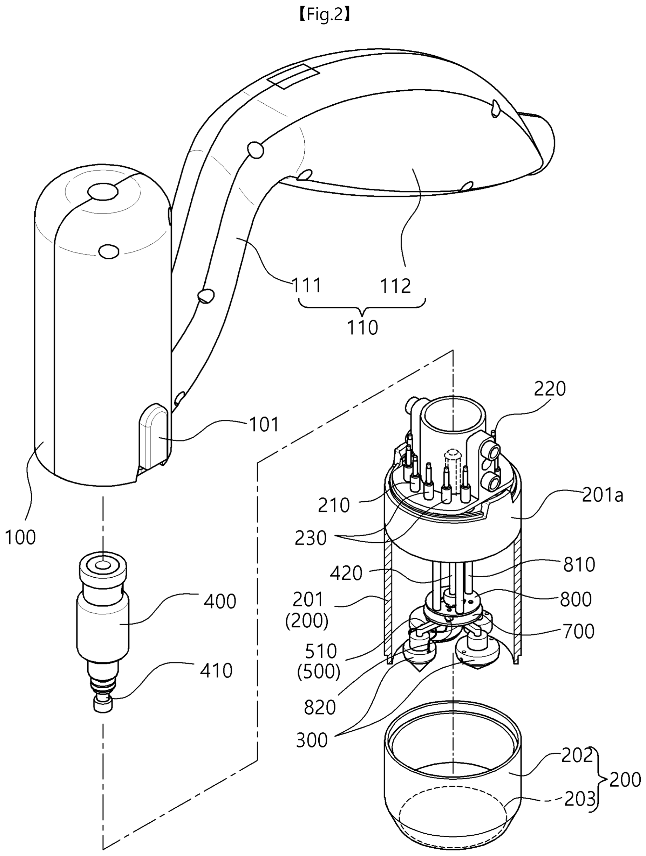

FIG. 1 is a perspective view showing an embodiment of an ultrasonic treatment handpiece according to the present disclosure and FIG. 2 is an exploded perspective view showing an embodiment of the ultrasonic treatment handpiece according to the present disclosure.

Referring to FIGS. 1 and 2, an ultrasonic treatment handpiece according to the present disclosure includes a body housing 100 to which an ultrasonic wave generating device is detachably coupled, and the ultrasonic wave generating device includes a cartridge housing 200, in which an ultrasonic transducer unit 300 is positioned, and emits ultrasonic waves to the skin through a window 203 disposed at a lower portion of the cartridge housing 200.

The ultrasonic treatment handpiece according to the present disclosure is connected to a control body 1000 through a handpiece cable assembly 1100, which includes a power cable, and a medium supply line and a medium discharge line for circulating a medium in the cartridge housing 200, and it should be noted that the handpiece cable assembly and the control body 1000 is a known example for known ultrasonic treatment devices and can be variously modified, so a detailed description thereof is omitted.

The ultrasonic wave generating device, that is, the cartridge housing 200, is detachably coupled to the body housing 100 and can be replaced after being used a preset number of times.

Further, the ultrasonic wave generating device includes an embodiment of an ultrasonic wave generating device having a plurality of ultrasonic transducers according to the present disclosure that will be described in detail below.

A cartridge locking portion 101 that can engage and fix the cartridge housing 200 is disposed on the body housing 100. The cartridge locking portion 101 is elastically supported by a spring, so a wedge-shaped engaging portion engages with a locking groove located inside the cartridge housing 200 and fixes the coupled state of the cartridge housing 200, and when pressed, the engaging portion is disengaged from the locking groove so that the cartridge housing 200 can be separated.

In addition to the cartridge lock 101, a known detachable locking structure is applied to the cartridge housing 200, whereby the cartridge housing 200 can be kept coupled with the may body housing 100 or separated, and accordingly, it should be noted that further detailed description is omitted.

A handle 110, which a practitioner can hold by hand, is disposed on one side of the may body housing 100.

The handle 110 includes a handle bridge 111 that bends upward from the body housing 100 and a handle body 112 that is curved downward from the handle bridge 111.

A practitioner can easily perform a procedure by holding the handle body 112 bending from the handle bridge 111 and integrally extending downward and by easily bringing the window 203 of the cartridge housing 200 into close contact the skin.

The handle 110 has a shape bending upward and then extending downward and is designed such that the portion extending downward, that is, the handle body 112 is held, so the load that is applied to a practitioner during a procedure can be minimized and it is possible to easily bring the window 203 of the cartridge housing 200 into close contact with the skin and perform a procedure.

Meanwhile, an ultrasonic wave generating device having a plurality of ultrasonic transducers according to the present disclosure includes the cartridge housing 200 having the window that is disposed on the bottom to come into contact with the skin and allow ultrasonic waves to pass through, and a plurality of ultrasonic transducer units 300 disposed inside the cartridge housing 200 and generating ultrasonic waves.

The ultrasonic wave generating device includes the cartridge housing 200 having the window 203 disposed at the lower portion to allow ultrasonic waves to pass through, and the ultrasonic transducer units 300 located inside the cartridge housing 200 and generating ultrasonic waves downward.

The window 203 is made of a transparent or translucent material through which ultrasonic waves pass, and is made of a known material that allows ultrasonic waves to pass through, so it should be noted that further detailed description is omitted.

The cartridge housing 200 includes an upper housing 201 that is detachably coupled to the body housing 100 and a housing-sealing cap 202 that is detachably coupled to the lower side of the upper housing 201.

The window 203 is formed on the bottom of the housing-sealing cap 202 and is sized to enable an ultrasonic procedure in contact with the patient's skin.

The cartridge housing 200 has a sealed internal structure and is filled with an ultrasonic transmission medium.

It is exemplified that the ultrasonic transmission medium is an insulating liquid, and the insulating liquid can be variously modified and implemented into well-known insulating liquids that are not electrically conductive, and thus, it should be noted that further detailed description is omitted.

The ultrasonic transmission medium not only serves to transmit ultrasonic waves but also serves to cool the patient's skin through the window 203 that comes into contact with the skin.

A cartridge board 201a that seals the inside of the cartridge housing 200 is disposed at the upper portion of the cartridge housing 200, and a medium supply protruding tube 210 and a medium discharge protruding tube 220 for circulating the ultrasonic transmission medium are disposed to protrude on the top of the cartridge board 201a.

A rotary motor unit 400 includes a rotary shaft 420 mounted on the top of a rotary mount 500, and it is exemplified that the rotary shaft 420 has a structure that is mounted to protrude at the center of the top of the rotary mount 500 and is detachably coupled to the rotary motor unit 400.

The rotary mount 500, on which the plurality of ultrasonic transducer units 300 are mounted, is rotatably disposed in the cartridge housing 200.

The rotary shaft 420 is disposed upright at the center inside the cartridge housing 200, with its lower end connected to the rotary mount 500 on which the plurality of ultrasonic transducer units 300 are mounted, and with a portion of its upper end protruding from the top of the cartridge board 201a and connected to the rotary motor unit 400.

The rotation motor unit 400 is connected to the rotary shaft 420 and transmits rotational force to the rotary shaft 420, thereby rotating the rotary mount 500 on which the plurality of ultrasonic transducer units 300 are mounted around the rotary shaft 500.

The plurality of ultrasonic transducer units 300 are mounted on the rotary mount 500 at eccentric positions, spaced apart from the rotation center of the rotary mount 500, that is, the rotation center connected to the rotary shaft 420.

The rotary mount 500 may include a plurality of mounting arm members 510 disposed radially from the rotation center to which the rotary shaft 420 is connected.

The plurality of ultrasonic transducer units 300 are respectively mounted at the ends of the plurality of mounting arm members 510, and may be disposed at equal distances from one another with respect to the rotation center of the rotary mount 500, or at different distances from one another with respect to the rotation center of the rotary mount 500.

In summary, all of the plurality of ultrasonic transducer units 300 may be positioned at equal distances from one another with respect to the rotation center of the rotary mount 500, or at least one of the ultrasonic transducer units 300 may be positioned at a different distance with respect to the rotation center of the rotary mount 500.

The plurality of mounting arm members 510 may be formed to have equal lengths or to have different lengths.

Further, the plurality of ultrasonic transducer units 300 may be ultrasonic transducers whose focal points reach the same position, that is, the same depth, or may be ultrasonic transducers whose focal points reach different positions, that is, different depths.

Further, some of the plurality of ultrasonic transducer units 300 may be ultrasonic transducers whose focal points reach the same position, that is, the same depth, while the remaining ultrasonic transducer units 300 of the plurality may be ultrasonic transducers whose focal points reach different positions, that is, different depths.

Further, the plurality of ultrasonic transducer units 300 are ultrasonic transducers whose focal points of ultrasonic waves reach the same position, that is, the same depth, but at least any one of them is mounted at a different height, so the focal point of at least any one ultrasonic wave may be located at a different depth.

The focal points of the ultrasonic waves generated from the plurality of ultrasonic transducer units 300 may be adjusted by adjusting the mounting heights of the plurality of ultrasonic transducer units 300.

In summary, the plurality of ultrasonic transducer units 300 may have a structure in which the focal points of ultrasonic waves are located at the same position, that is, at the same depth, and the plurality of ultrasonic transducer units 300 may have a structure in which the focal point of the ultrasonic wave of at least any one of them is located at a different position, that is, at a different depth.

The bottoms of the plurality of ultrasonic transducer units 300, through which ultrasonic waves are emitted, are positioned perpendicularly to the window 203, so ultrasonic waves are emitted perpendicularly to the window 203.

The rotary motor unit 400 is disposed inside the body housing 100, and when the cartridge housing 200 is coupled to a lower portion of the body housing 100, the portion of the rotary shaft 420 protruding upward from the cartridge housing 200, that is, the portion protruding from the top of the cartridge board 201a, is connected to the rotary motor unit 400.

It is exemplified that the rotary motor unit 400 is disposed inside the body housing 100, and it is connected to the rotary shaft 420 when the cartridge housing 200 is coupled to the lower portion of the body housing 100, so when the lifespan of the ultrasonic transducer units 300 is reached, the cost for replacing the cartridge housing 200 can be reduced.

Though not shown, it should be noted that the rotary motor unit 400 may be mounted in the cartridge housing 200 and may be configured such that it is replaced together when the lifespan of the ultrasonic transducer units 300 are reached and the cartridge housing 200 is replaced.

The rotary shaft 420 is disposed to protrude from the top of the cartridge board 201a and is rotatably disposed.

The rotary shaft 420 is rotatably disposed through the cartridge board 201a and can be achieved using a well-known sealing structure that seals a rotary shaft, so it should be noted that further detailed description is omitted.

The rotary shaft 420 has a connecting shaft portion 421 protruding upward from the cartridge board 201a, that is, the cartridge housing 200 and connected to the rotary motor unit 400, and a shaft adapter portion 410 in which the connecting shaft portion 421 is inserted to be connected to it is disposed on the shaft of the rotary motor unit 400.

It is exemplified that the shaft adapter portion 410 has a shaft insertion portion open downward such that the connecting shaft portion 421 is inserted therein, the connecting shaft portion 421 is a shaft having a polygonal cross-section, and the shaft insertion portion is a polygonal insertion groove corresponding to the connecting shaft portion 421.

A supply tube connecting portion (not shown) and a discharge tube connecting portion (not shown), which connect the medium supply protruding tube 210 and the medium discharge protruding tube 220 to a medium circulator (not shown) disposed in the control body 1000 controlling operation of the ultrasonic treatment handpiece when the cartridge housing 200 is coupled, are disposed in the body housing 100.

The control body 1000 may be variously modified in well-known ultrasonic treatment devices including a controller that controls operation of an ultrasonic treatment handpiece and a medium circulator that circulates an ultrasonic transmission medium, so it should be noted that further detailed description is omitted.

Though not shown, the medium circulator may be variously modified into well-known cooling water circulation structure including a medium storage tank, a medium supply line connecting the medium storage tank and a supply tube connector 120, a medium discharge line connecting the medium storage tank and a discharge tube connector 130, a valve disposed in the medium supply line, and a medium cooler disposed in the medium storage tank. Accordingly, it should be noted that further detailed description is omitted.

The supply tube connector (not shown) includes a first protruding tube insertion portion in which the medium supply protruding tube 210 is inserted, and the discharge tube connector (not shown) includes a second protruding tube insertion portion in which the medium discharge protruding tube 220 is inserted.

It is exemplified that the medium supply protruding tube 210 is inserted in the first protruding tube insertion portion and is connected to the medium supply line when the channel is opened, and the medium discharge protruding tube 220 is inserted in the second protruding tube insertion portion and is connected to the medium discharge line when the channel is opened.

It should be noted that the medium supply protruding tube 210 and the supply tube connector (not shown), and the medium discharge protruding tube 220 and the discharge tube connector (not shown) may be variously modified into well-known tube connection structures connecting two tubes and including a valve that is opened when the two tubes are connected.

When the cartridge housing 200 is coupled to the body housing 100, the connecting shaft portion 421 of the rotary shaft 420 is inserted into the shaft insertion portion of the shaft adapter portion 410, whereby the rotary shaft 420 is connected to the shaft of the rotary motor unit 400, the medium supply protruding tube 210 is inserted into the first protruding tube insertion portion of the supply tube connector 120 and is connected to the medium circulator of the control body 1000, and the medium discharge protruding tube 220 is inserted into the second protruding tube insertion portion of the discharge tube connector 130 and is connected to the medium circulator of the control body 1000.

Further, a pair of main power supply terminals 230 for supplying power to the ultrasonic transducer units 300 is provided on the top of the cartridge housing 200, that is, the top of the cartridge board 201a, and though not shown, second main power supply terminals to which the pair of main power supply terminals 230 is connected and that is connected to the control body 1000 through the handpiece cable assembly 1100 are disposed in the body housing 100.

It is exemplified that the first main power supply terminals 230 are protruding terminals that protrude from the top of the cartridge housing 200, that is, the top of the cartridge board 201a, and the second main power supply terminals are insertion-type terminals in which the first main power supply terminals 230 can be inserted and connected.

When the cartridge housing 200 is coupled to the body housing 100, the pair of first main power supply terminals 230 is inserted into the pair of second main power supply terminals and they are connected to each other, whereby the ultrasonic transducer units 300 are connected to the control body 1000 through the handpiece cable assembly 1100, and accordingly, power can be supplied from the control body 1000 and the operation can be controlled.

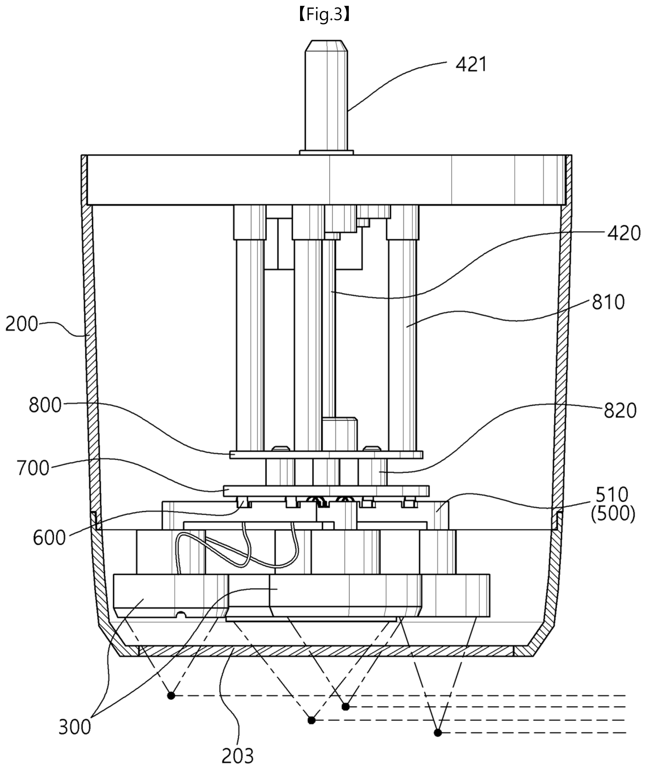

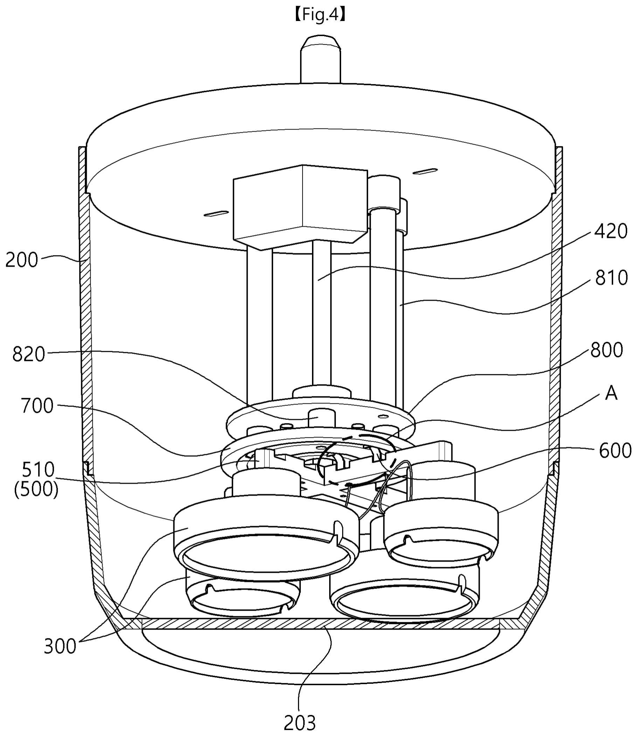

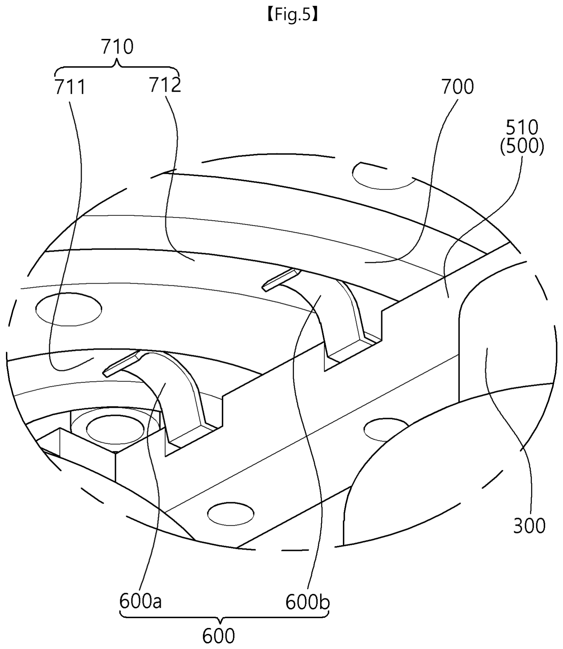

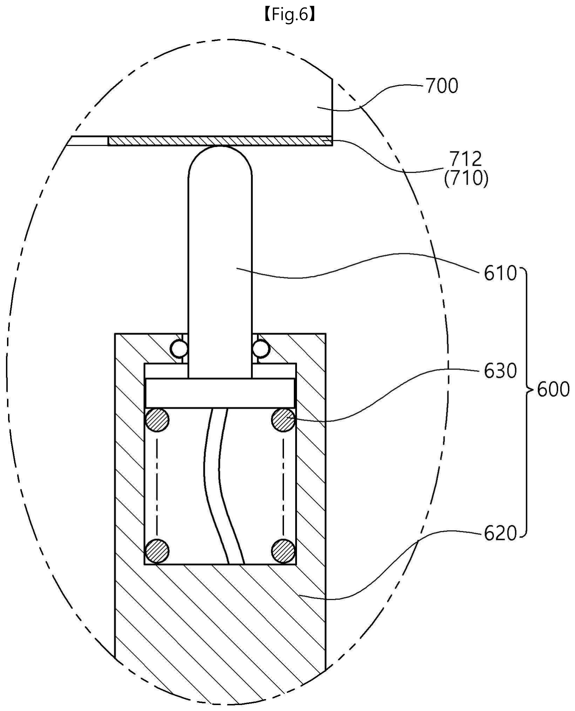

Meanwhile, FIG. 3 is a cross-sectional view showing an embodiment of an ultrasonic wave generating device having a plurality of ultrasonic transducers according to the present disclosure, FIG. 4 is a partial enlarged bottom perspective view showing an embodiment of the ultrasonic wave generating device having a plurality of ultrasonic transducers according to the present disclosure, FIG. 5 is an enlarged view of the portion A of FIG. 4, and FIG. 6 is a cross-sectional view showing another embodiment of the portion A of FIG. 4.

An embodiment of an ultrasonic generating device having a plurality of ultrasonic transducers according to the present disclosure is described hereafter in detail with reference to FIG. 2 to FIG. 6.

In an embodiment of an ultrasonic generating device having a plurality of ultrasonic transducers according to the present disclosure, the ultrasonic transducer units 300 are mounted at eccentric positions on the rotary mount 500 that is rotated by the rotary motor unit 400, so that when the rotary mount 500 rotates, the focal points of the ultrasonic waves generated from the ultrasonic transducer units 300 move along circular paths, i.e., along circles having a predetermined radius from the rotational axis of the rotary motor unit 400.

The plurality of ultrasonic transducer units 300 are mounted at eccentric positions on a rotary mount 500 that is rotated by the rotary motor unit 400 and rotate in a plane, whereby the focal points of the ultrasonic waves generated from the ultrasonic transducer units 300 move along circular paths in the same plane.

Each of the ultrasonic transducer units 300 moves along a circular path having a radius corresponding to its distance from the rotation center of the rotary mount 500, whereby the focal points of the ultrasonic waves generated from the ultrasonic transducer units 300 move along circular paths in the same plane.

The ultrasonic transducer units 300 are rotated around the rotary shaft 420 while the ultrasonic generation surfaces, from which the ultrasonic waves are generated, are positioned in parallel, i.e., the ultrasonic generation surfaces are positioned horizontally, in the direction perpendicular to the window 203, whereby the focal points of the ultrasonic waves generated from the ultrasonic transducer units 300 move along circular paths in the same plane.

That is, the ultrasonic wave generating device having a plurality of ultrasonic transducers according to the present disclosure moves each of the focal points of the ultrasonic waves generated from the plurality of ultrasonic transducer units 300 at a predetermined radius in the same plane while positioning them at a uniform depth in the skin to form circular paths in the plane, and whereby it is possible to further enhance treatment performance by uniformly and evenly applying energy within the radius.

Further, according to the ultrasonic wave generating device having a plurality of ultrasonic transducers according to the present disclosure, the plurality of ultrasonic transducer units 300 mounted eccentrically on the rotary mount 500 are horizontally positioned and rotated around the rotary shaft 420, so the movement paths of the focal points are formed in circular shapes in a plane. Accordingly, it is easy to adjust the output of the ultrasonic waves and it is possible to selectively design the radius of movement of the focal points to be narrower or wider.

Further, all of the plurality of ultrasonic transducer units 300 are disposed at the same distance from the rotation center of the rotary mount 500, or at least one is disposed at a different distance from the rotation center of the rotary mount 500, whereby it is possible to vary the rotation radius and variously set the treatment area.

Further, the plurality of ultrasonic transducer units 300 may have a structure in which the focal points of ultrasonic waves are located at the same position, that is, at the same depth, and the plurality of ultrasonic transducer units 300 may have a structure in which the focal point of the ultrasonic wave of at least any one of them is located at a different position, that is, at a different depth, so the depths that the focal points of the ultrasonic waves reach are different, whereby it is possible to achieve a treatment effect on the skin at various depths.

Meanwhile, an embodiment of the ultrasonic wave generating device having a plurality of ultrasonic transducers further includes a plurality of power supply protruding terminal units 600 that protrude upward from the rotary mount 500 and supply power to the ultrasonic transducer units 300, and a power supply board 700 that is spaced above the rotary mount 500 in the cartridge housing 200 and to which the plurality of power supply protruding terminal units 600 are connected.

The plurality of power supply protruding terminal units 600 are respectively provided on the plurality of mounting arm members 510 as a pair of a positive terminal and a negative terminal, and are connected by wires to the respective ultrasonic transducer units 300 mounted on the mounting arm members 510, thereby applying electric power to the ultrasonic transducer units 300.

A ring-shaped electrode pattern 710, which is connected through a power cable and to which power is applied, is disposed on the bottom of a power supply board 700, and when the rotary mount 500 is rotated around the rotary shaft 420 by a rotary motor unit 400, the power supply protruding terminal units 600 are moved along a circular path with the upper ends thereof in contact with a circular pattern for power supply, whereby power can be stably supplied to the ultrasonic transducer units 300.

The ring-shaped electrode pattern 710 is electrically connected to the first main power supply terminals 230, which protrude from the top of the cartridge housing 200, that is, the top of the cartridge board 201a, by power cables through terminals disposed through the power supply board 700.

Accordingly, when the cartridge housing 200 is coupled to the body housing 100, the pair of first main power supply terminals 230 are inserted into the pair of second main power supply terminals and they are connected to each other, whereby the ultrasonic transducer units 300 are connected to the control body 1000 through the handpiece cable assembly 1100, and accordingly, power can be supplied from the control body 1000 and the operation can be controlled.

Further, it is exemplified that the power supply protruding terminal units 600 are elastic brush terminals for electrical connection, which are bent in a semicircular shape so that connection is elastically made at a curved surface portion. The power supply protruding terminal units 600 are kept in elastic contact with the ring-shaped electrode pattern 710, so they can be stably connected to the ring-shaped electrode pattern 710 while moving along a circular path.

The power supply protruding terminal unit 600 includes a negative protruding terminal unit 600a and a positive protruding terminal unit 600b, which are spaced at different distances from the rotary shaft 420, and the ring-shaped electrode pattern 710 includes a first electrode pattern 711 with a radius corresponding to the rotation radius of the negative protruding terminal unit 600a and a second electrode pattern 712 with a radius corresponding to the rotation radius of the positive protruding terminal unit 600b.

The negative protruding terminal unit 600a and the positive protruding terminal unit 600b are in contact with the first electrode pattern 711 and the second electrode pattern 712, which have different radii, respectively, and are maintained in an elastic contact state, whereby they move along a circular path in contact with the ring-shaped first and second electrode patterns 711 and 712, respectively, and accordingly, power can be stably supplied to the ultrasonic transducer units 300.

Each ultrasonic transducer unit 300 is electrically connected to the control body 1000 through the power supply protruding terminal unit 600, and its operation is controlled by the control body 1000.

The control body 1000 may operate all of the plurality of ultrasonic transducer units 300 or may selectively operate at least any one of the plurality of ultrasonic transducer units 300.

Further, since the ultrasonic transmission medium is an insulating liquid, even if the power supply protruding terminal units 600 are exposed within the cartridge housing 200, a short circuit does not occur, and power can be stably supplied into the ultrasonic transducer units 300 with the negative protruding terminal unit 600a and the positive protruding terminal unit 600b in contact with the first electrode pattern 711 and the second electrode pattern 712 that are the two exposed ring-shaped electrode patterns.

Further, an embodiment of the ultrasonic wave generating device having a plurality of ultrasonic transducers according to the present disclosure may further include: a board position-fixing board 800 that is disposed between the cartridge board 201a and the power supply board 700 and supports the position of the power supply board 700; a plurality of first board fixing portions 810 that have both ends fixed to the cartridge board 201a and the board position-fixing board 800 and that support the position of the board position-fixing board 800; and a plurality of second board fixing portions 820 that have both ends fixed to the board position-fixing board 800 and the power supply board 700 and that support the position of the power supply board 700.

The rotary shaft 420 is connected at an eccentric position on the top of the ultrasonic transducer units 300 through the board position-fixing board 800 and the power supply board 700.

The board position-fixing board 800 is fixed in position by being connected to the top of the cartridge housing 200, that is, to the cartridge board 201a, by the plurality of first board fixing portions 810, and the power supply board 700 is stably fixed in position by being connected to the board position-fixing board 800 by the plurality of second board fixing portions 820.

The plurality of first board fixing portions 810 and the plurality of second board fixing portions 820 are formed in a hollow tubular shape, and power cables connecting the ring-shaped electrode pattern 710 and the first main power supply terminal 230 can be disposed inside them.

The power cable connecting the ring-shaped electrode pattern 710 and the first main power supply terminal 230 may not be exposed to the outside by being disposed through the insides of the plurality of first board fixing portions 810 and the plurality of second board fixing portions 820.

FIG. 6 is a cross-sectional view showing another embodiment of the portion A of FIG. 4 and showing another embodiment of the power supply protruding terminal unit 600.

Referring to FIG. 6, the power supply protruding terminal unit 600 includes a jig pin member 610 that is connected to the ring-shaped electrode pattern 710 and is electrically connected to the ultrasonic transducer unit 300 through a wire, a pin insertion member 620 that is disposed on the top of the ultrasonic transducer unit 300 such that the jig pin member 610 can be moved up and down, and a pin support spring member 630 that is disposed in the pin insertion member 620 and elastically supports the jig pin member 610.

The jig pin member 610 is elastically supported by the pin support spring member 630, with the upper end thereof in contact with the ring-shaped electrode pattern 710.

When the plurality of ultrasonic transducer units 300 are eccentrically rotated around the rotary shaft 420 together with the rotary mount 500 by the rotary motor 400, the jig pin member 610 elastically supported by the pin support spring member 630 with the upper end in contact with the ring-shaped electrode pattern 710 moves along a circular path along the ring-shaped electrode pattern 710, whereby power can be stably supplied to the ultrasonic transducer units 300.

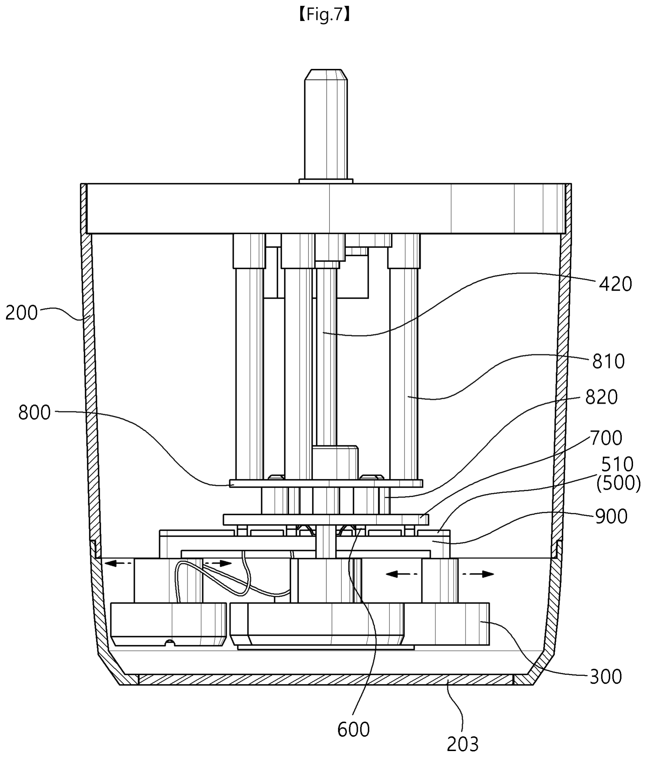

FIG. 7 is a partial enlarged view showing another embodiment of the ultrasonic wave generating device having a plurality of ultrasonic transducers according to the present disclosure. Referring to FIG. 7, another embodiment of the ultrasonic wave generating device having a plurality of ultrasonic transducers according to the present disclosure further includes an ultrasonic transducer moving unit 900 that is disposed under the rotary mount 500 and adjusts the distance between the rotation center of the rotary mount 500 and the ultrasonic transducer units 300 by linearly moving the ultrasonic transducer units 300.

The ultrasonic transducer moving unit 900 is disposed on the bottom of the rotary mount 500, that is, the bottom of the mounting arm member 510, and linearly moves the ultrasonic transducer units 300 in the circumferential direction from the rotation center of the rotary motor unit 400, thereby being able to adjust the rotation radius of the ultrasonic transducer units 300 with respect to the rotation center of the rotary motor unit 400.

It is exemplified that the power supply protruding terminal units 600 are mounted on the top of the ultrasonic transducer moving unit 900, that is, the top of the mounting arm member 510 having the ultrasonic transducer moving unit 900 mounted on its bottom.

The ultrasound transducer units 300 are linearly moved by the ultrasonic transducer moving unit 900, whereby the radius of rotation that is performed by the rotary shaft 420 can be adjusted.

That is, another embodiment of the ultrasonic wave generating device according to the present disclosure allows the ultrasound transducer units 300 to be moved bidirectionally in a straight direction in a plane passing through the rotation center of the rotary mount 500, thereby allowing the movement radius of the focal points, which are formed in a circular shape on the plane, to be adjusted in various ways.

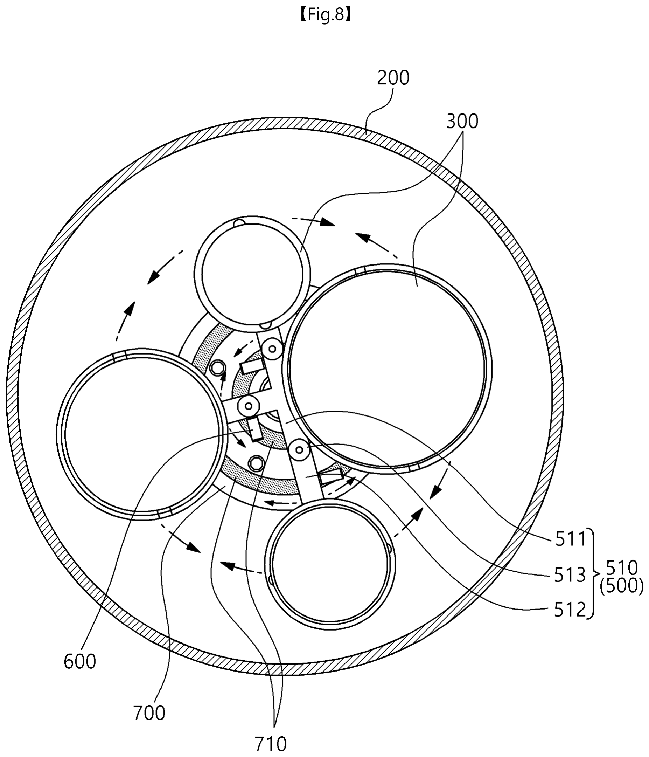

Further, FIG. 8 is a cross-sectional view showing another embodiment of the ultrasonic wave generating device having a plurality of ultrasonic transducers according to the present disclosure. Referring to FIG. 8, the mounting arm member 510 may include a first arm member 511 connected to the rotary shaft 420, a second arm member 512 rotatably hinged to the first arm member 511 and on which the ultrasound transducer units 300 are mounted, and an arm rotation motor 513 rotating the second arm member 512 in a plane about the hinge.

The mounting arm member 510 rotates the second arm member 512 about the hinge in a plane using the arm rotation motor 513, and can rotate it within a preset radius.

While the first arm member 511 is rotated around the rotary shaft 420 by the operation of the rotary motor unit 400, the mounting arm member 510 rotates the second arm member 512, on which the ultrasonic transducer units 300 are mounted, using the arm rotation motor 513, whereby it is possible to diversify the movement pattern of focal points of ultrasonic waves, and accordingly, it is possible to enhance the ultrasonic treatment or skin care effect. Further, the movement patterns of the focal points of ultrasonic waves are implemented in various ways to be suitable for the target skins so that appropriate procedures can be performed in accordance with the skin conditions.

The present disclosure selectively generates a plurality of ultrasonic waves using a plurality of ultrasonic transducers, and allows the focal points of ultrasonic waves to penetrate to different depths inside the skin or individually controls the intensities of ultrasound waves, thereby being able to achieve an ultrasonic treatment or skin care effect in accordance with the skin conditions of patients and to enhance the ultrasound treatment or skin care effect.

Further, the present disclosure applies energy uniformly and evenly to a treatment area by moving the focal points of ultrasonic waves in a plane at a uniform depth inside the skin, and applies energy uniformly and evenly within a predetermined radius by forming the focal points of the ultrasonic waves at a uniform depth inside the skin in a circular shape having the radius, thereby being able to improve treatment performance.

The present disclosure can further improve the treatment effect by moving the focal points of ultrasonic waves along circular paths in a plane by rotating the ultrasonic transducer units 300 at offset positions in the plane to make it easy to adjust the output of ultrasonic waves, and can increase the versatility of the treatment by expanding the range of the movement radius of the focal points.

Further, the present disclosure can minimize the load on the motor and reduce manufacturing costs by simplifying the structure for moving the focal points of the ultrasonic waves generated by the plurality of ultrasonic transducer units 300 along circular paths in the same plane.

The present disclosure is not limited to the embodiments described above and may be modified in various ways without departing from the scope of the present disclosure and the modifications should be construed as being included in the present disclosure.

## Claims

1. An ultrasonic wave generating device having a plurality of ultrasonic transducers, the ultrasonic wave generating device comprising:
a cartridge housing having, on a bottom thereof, a window configured to come into contact with a skin and allow ultrasonic waves to pass through;
a plurality of ultrasonic transducer units disposed in the cartridge housing and configured to generate ultrasonic waves;
a rotary mount on which the plurality of ultrasonic transducer units are mounted and which is rotatably disposed in the cartridge housing; and
a rotary motor unit configured to rotate the rotary mount.

2. The ultrasonic wave generating device of claim 1, wherein the rotary motor unit comprises a rotary shaft mounted on the rotary mount and connected to the rotary motor unit, and
the plurality of ultrasonic transducer units are mounted on the rotary mount at positions spaced apart from and eccentric to a rotation center of the rotary mount.

3. The ultrasonic wave generating device of claim 2, wherein the rotary mount comprises a plurality of mounting arm members disposed radially from a rotation center to which the rotary shaft is connected.

4. The ultrasonic wave generating device of claim 2, wherein all of the plurality of ultrasonic transducer units have structures that position focal points of ultrasonic waves at the same depth, or at least any one of the plurality of ultrasonic transducer units has a structure that positions the focal point of an ultrasonic wave at a different depth.

5. The ultrasonic wave generating device of claim 2, wherein the plurality of ultrasonic transducer units are all disposed to be spaced apart from the rotation center of the rotary mount by the same distance, or at least any one of the plurality of ultrasonic transducer units is disposed to be spaced apart from the rotation center of the rotary mount by a different distance.

6. The ultrasonic wave generating device of claim 2, wherein the ultrasonic transducer unit is rotated around the rotary shaft while an ultrasonic generation surface is positioned horizontally, thereby moving a focal point of the ultrasonic wave generated from the ultrasonic transducer unit along a circular path in the same plane.

7. The ultrasonic wave generating device of claim 2, further comprising:
a plurality of power supply protruding terminal units protruding upward from the rotary mount and configured to supply power to the plurality of ultrasonic transducer units; and
a power supply board spaced above the rotary mount in the cartridge housing and to which the plurality of power supply protruding terminal units are connected.

8. The ultrasonic wave generating device of claim 7, wherein a ring-shaped electrode pattern connected through a power cable to receive power is disposed on a bottom of the power supply board, and
the power supply protruding terminal units move along a circular path along the ring-shaped pattern while elastically in contact with the ring-shaped electrode pattern.

9. The ultrasonic wave generating device of claim 7, wherein the cartridge housing has a sealed internal structure and is filled with an ultrasonic transmission medium, and
the ultrasonic transmission medium is an insulating liquid.

10. The ultrasonic wave generating device of claim 8, further comprising:
a board position-fixing board disposed between a top of the cartridge housing and the power supply board and configured to support the position of the power supply board;
a plurality of first board fixing portions having both ends fixed to the top of the cartridge housing and the board position-fixing board and configured to support the position of the board position-fixing board; and
a plurality of second board fixing portions having both ends fixed to the board position-fixing board and the power supply board and configured to support the position of the power supply board.

11. The ultrasonic wave generating device of claim 10, wherein the plurality of first board fixing portions and the plurality of second board fixing portions are formed in a hollow tubular shape, and power cables connecting the ring-shaped electrode pattern and a first main power supply terminal protruding upward from the cartridge housing are disposed inside the first and second board fixing portions.

12. The ultrasonic wave generating device of claim 2, further comprising
an ultrasonic transducer moving unit disposed under the rotary mount and configured to adjust the distance between the rotation center of the rotary mount and the ultrasonic transducer units by linearly moving the ultrasonic transducer units.

13. The ultrasonic wave generating device of claim 3, wherein the mounting arm members include:
a first arm member connected to the rotary shaft;
a second arm member rotatably hinged to the first arm member and on which the ultrasound transducer units are mounted; and
an arm rotation motor configured to rotate the second arm member in a plane about the hinge.

14. A therapeutic ultrasonic wave generating device comprising:
a body housing connected to a control body through a handpiece cable assembly; and
an ultrasound generating device detachably coupled to the body housing,
wherein the therapeutic ultrasonic wave generating device is the ultrasonic wave generating device having a plurality of ultrasonic transducers of any one of claims 1 to 13.
